# EUROPEAN PATENT APPLICATION

(11) **EP 4 166 535 A1**
(43) Date of publication of application: **19.04.2023**
(21) Application number: 20940528.1
(22) Date of filing: 15.06.2020
(51) Int. Cl.: C07C 231/22, A23L 27/10

(54) **METHOD FOR STABILIZING SANSHOOLS**

(71) Applicant: Yamamoto, Yoshie, Kainan-shi, Wakayama 642-0024 (JP); Wakayama University, Wakayama-shi, Wakayama 640-8510 (JP)
(72) Inventor: MITANI, Takahiko, Wakayama-shi, Wakayama 640-8510 (JP); YAMAMOTO, Nami, Wakayama-shi, Wakayama 640-8510 (JP); YAHATA, Yasuko, Wakayama-shi, Wakayama 640-8510 (JP); TSUCHIDA, Takashi, Kainan-shi, Wakayama 642-0024 (JP)
(74) Representative: Vossius & Partner Patentanwälte Rechtsanwälte mbB
(86) International application number: PCT/JP2020/025115
(87) International publication number: WO 2021/255943

(57) **Abstract**

A method for stabilizing sanshools, the method including Step A and Step B below:
Step A: obtaining a sanshool solution containing stabilizing component by adding a stabilizing component of sanshools to a sanshool-containing liquid and;
Step B: obtaining an emulsion by adding, to the sanshool solution containing stabilizing component, an emulsifier and a solvent that is immiscible with the sanshool solution containing stabilizing component.

## Description

### Technical field

The present invention relates to a method for stabilizing sanshools, which are pungent components of sansho (Zanthoxylum piperitum).

### Background

Sansho contains a pungent component with a numbing sensation, and it is known that this pungent component comprises sanshools such as hydroxy-α-sanshool, which is an alkylamide derivative of long-chain unsaturated fatty acids contained in sansho (Patent Document 1). It is also known that an extract from sansho is used for foods, cosmetics, pharmaceuticals, etc. (Patent Documents 1 and 2).

### Prior art

1. JP2013-103901A
2. JP2001-354958A

### Problems to be solved by the invention

When sansho is used as an ingredient of products such as foods, cosmetics, and pharmaceuticals, the fruit and pericarp of sansho are sometimes pulverized or extracted. Although taste, aroma, color, and other flavors of sansho are maintained relatively stable in the dry state of fruit and pericarp of sansho. However, when pericarp of sansho is powdered, sanshools contained therein decompose and decrease rapidly. Sanshools extracted from this powder and diluted with a liquid or pulverization thereof, the sanshools contained therein are also rapidly decomposed and reduced. Therefore, products containing powders or extracts of sansho pericarp may deteriorate in quality, and it is required to stabilize sanshools and suppress deterioration.

The inventor found components which stabilize sanshools (hereinafter referred to as "stabilizing component") in order to prevent deterioration of sanshools. These include tocopherols, sodium benzoate, sodium ascorbate, and the like. (JP 6630880)

Hence, a certain degree of success has been achieved in stabilization of sanshools, but in order to further advance their application to commercial products, liquid or powdery compositions in which sanshools and stabilizing components coexist, is required.

Namely, sanshools are kept stable in extracts obtained by extracting sansho pericarp powder with ethanol alone or with ethanol to which the stabilizing components have been added. When it is diluted with water or the like, or powdered with dextrin or the like, the content of sanshools rapidly decreases in any liquid formulation or powder under normal room temperature conditions. In addition, sanshools are kept stable in the extract of sansho pericarp powder in the edible oil alone or in the edible oil to which the stabilizing component is added. Contrarily, the content of sanshools powdered with dextrin rapidly decreases when placed under normal room temperature. It is considered to be because of the separation of the sanshools and the stabilizing component due to dilution or pulverization.

Thus, no composition to stabilize sanshools in a liquid or powdery state or method for producing the same has been found so far. The purpose of the present invention is to provide a composition in a liquid or powdery state in which sanshools are stabilized, and is to provide a method for producing the same.

### Means to solve problems

The present invention provides the stabilization method using an emulsion of sanshools as follows. Generally, types of emulsions include oil-in-water (hereinafter "o/w"), water-in-oil (hereinafter "w/o"), and water-in-oil-in-water (hereinafter "w/o/w") are known.

Firstly, the present invention is a method for stabilizing sanshools, the method including: a step (step A) for adding a stabilizing material of sanshools to a sanshool-containing liquid and obtaining a stabilizing material-containing sanshool solution; and a step (step B) for obtaining an emulsion by adding, to the stabilizing material-containing sanshool solution, an emulsifier and a solvent that is immiscible with the stabilizing material-containing sanshool solution.

Secondly, the present invention provides the method for stabilizing sanshools described in item 1 above, wherein in Step A above, an oil-soluble stabilizing component of sanshools is added to the oil extract of sansho to obtain a sanshool solution containing a stabilizing component, then in Step B above, an emulsifier and water or a buffer solution are added to the said sanshool solution containing the stabilizing component to obtain an o/w emulsion.

Thirdly, the present invention provides the method for stabilizing sanshools described in item 1 above, wherein in Step A above, a stabilizing component of sanshools which soluble in water or ethanol is added to the ethanol extract of sansho to obtain a sanshool solution containing a stabilizing component, then in Step B above, an emulsifier and an edible oil are added to the sanshool solution containing the stabilizer to obtain a w/o emulsion.

Fourth, the present invention provides the method for stabilizing sanshools described in item 1, wherein in Step A, water- or ethanol-soluble sanshool-stabilizing component is added to the ethanol extract of sansho to obtain a sanshool solution containing a stabilizing component, then in Step B, an emulsifier and an edible oil are added to the sanshool solution containing a stabilizing component above to obtain a w/o emulsion. Further in Step B, emulsifier which is the same with or different from the said emulsifier to the w/o emulsion above and water or buffer containing the ethanol-soluble stabilizer to obtain a w/o/w emulsion.

Fifth, the present invention provides the method for stabilizing sanshools including the steps wherein an emulsion obtained by the method for stabilizing sanshools described in item 1, 2, 3 or 4 is mixed with saccharide powder with a dextrose equivalent value (dextrose equivalent, hereinafter referred to as "DE value") 25 or more, insoluble polysaccharide, precipitated silica, or a combination thereof, and pulverized by vacuum drying or freeze-drying.

In the present invention, the liquid containing sanshools is a sansho extract, which is obtained by extracting sansho with an edible oil or at least one of a water-soluble organic solvent and a water-containing organic solvent. The extraction operation with a water-soluble organic solvent and a water-containing organic solvent is carried out under the condition that pH is 6 or more. The liquid containing synthesized sanshools can also be used.

The stabilizing components used in the present invention is one or more selected from the group consisting of tocopherols, dihydroxybenzoic acids or salts or esters thereof, trihydroxybenzoic acids or salts or esters thereof, hydroxycinnamic acids or salts thereof, ascorbic acids or salts or esters thereof, thiols, lipoic acids, flavonoids and the like.

The emulsifiers used in the present invention include sucrose fatty acid esters, ascorbic acid fatty acid esters, sorbitan fatty acid esters, lecithins, monoglycerides, and other emulsifiers permitted for use in the production of foods and pharmaceuticals. In the emulsification treatment, a water-soluble or fat-soluble stabilizing component, or an inclusion compound such as cyclodextrin, a chelating agent, a pH adjuster, or the like may be used in combination.

### Effect of the invention

According to the present invention, it is possible to provide a method for stabilizing sanshools, which can stabilize sanshools in sansho extract in a liquid or powder state.

### Embodiment of the invention

### (Method for emulsifying sanshools)

One embodiment of the present invention includes an emulsification step of emulsifying a sanshools solution containing a stabilizing component and an emulsifier in a solvent immiscible with the sanshools solution containing the stabilizing component. Yet another embodiment includes mixing these emulsions with suitable excipients and powdering by vacuum drying or freeze drying. According to the present invention, it becomes easier to stably store sanshools for a long period of time, and when sanshools are used in various products such as foods, supplementary foods, beverages, pharmaceuticals, quasi-drugs, cosmetics, feeds, detergents, and daily necessities, the sanshools can be present in these products in a stable state, so that the life of these products can be extended.

### (Sanshools)

The sanshools may be extracted from sansho or may be synthesized. Sanshools of the present embodiment include α-sanshool, β-sanshool, γ-sanshool, δ-sanshool, hydroxy-α-sanshool, hydroxy-β-sanshool, and hydroxy-γ-sanshool. Among these, sanshool is preferably hydroxy-α-sanshool and/or α-sanshool, which components abundantly contained in sansho.

Sanshools are not only known as pungent ingredients in foods, but also known to be used for pharmaceuticals, supplements, etc. for the purpose of enhancing salty taste, promoting saliva secretion, improving gastrointestinal motility (improvement of gastrointestinal paralysis (postoperative ileus)), anesthetic effect, memory promoting effect, fat burning effect. The use of sanshools stabilized by the method for stabilizing sanshools of the present embodiment is expected to improve the shelf life of various products containing sanshools.

The sanshools may be contained in a sansho extract extracted from sansho. The sansho extract is an extract extracted from the part of sansho that contains at least sanshools. The sansho extract may contain extracts other than sanshools extracted from sansho. The sansho extract may be present in the solvent used for extracting the sansho extract such as sanshools, or may be present in a concentrate obtained by removing at least part of this solvent. Alternatively, it may be a dried product obtained by removing the solvent by drying.

The sansho that can be used to obtain the sansho extract is not particularly limited as long as it is a plant belonging to the genus Zanthoxylum of the Rutaceae family. Specific examples of Sansho include Zanthoxylum piperitum DC, Zanthoxylum piperitum DC. var. inerme Makino, Zanthoxylum piperitum DC. forma brevispinosum Makino, Zanthoxylum schinifolium Sieb. et Zucc., Zanthoxylum bungeanum Maxim., Zanthoxylum armatum var. subtrifoliatum, and Zanthoxylum nitidum. Among these, Zanthoxylum piperitum DC (sansho)) and Zanthoxylum piperitum DC. var. inerme Makino (asakurazansho), which are commonly used in foods and have excellent quality and high safety, are preferable. Especially, a variety of Zanthoxylum piperitum DC. var. inerme Makino (asakurazansho), Budoh sansho which is a specialty of Wakayama Prefecture, is more preferred.

### (Method of obtaining sansho extract)

The part of sansho used to obtain the sansho extract may be any part of sansho, such as fruit, pericarp, seed, leaf, trunk, branch, root, and stem. From the viewpoint of the efficiency and workability of collection of sanshools, it is preferable to use fruits, pericarps, and leaves of sansho. Is more preferable to use pericarps of sansho.

The extraction method for obtaining sansho extract from the parts of sansho is not particularly limited as long as it is a conventionally-known method. For example, compression method, steam distillation method, solvent extraction method, supercritical extraction method, carbon dioxide gas extraction method, and oil adsorption method can be used. Among these methods, solvent extraction method is preferable because of the efficiency of extract of sanshools from sansho.

In the solvent extraction method, the parts of sansho are dried, cut, crushed, pulverized as needed, and then edible oil, organic solvent, water, or mixture of water and organic solvent are used as the extraction solvent. Examples of the organic solvent include the organic solvents described below. As the extraction solvent, a safe solvent that can be used for foods and pharmaceuticals is preferable.

If sanshools are obtained by synthesis, they may be synthesized according to a known synthesis method.

### (Stabilizing component)

Stabilizing components are the components indicated above, particularly safe stabilizing components that can be used in foods and pharmaceuticals are preferred. The stabilizing component in this embodiment is a stabilizing component suitable for the type of emulsion, and may be water-soluble or fat-soluble.

Water-soluble stabilizing components include dihydroxybenzoic acid such as protocatechuic acid or salts thereof; trihydroxybenzoic acid such as gallic acid or salts thereof; hydroxycinnamic acids such as caffeic acid and ferulic acid or salts thereof; ascorbic acids such as ascorbic acid and erythorbin or salts thereof; thiols such as thiourea, cysteine and glutathione; lipoic acids such as α-lipoic acid, dihydrolipoic acid, lipoamide and dihydroipoamide. Salts of these acids include alkali metal salts and alkaline earth metal salts, including sodium salts, potassium salts, magnesium salts and calcium salts.

Fat-soluble stabilizing components include esters of dihydroxybenzoic acid; esters of trihydroxybenzoic acid; ascorbic acid fatty acid esters; tocopherols such as α-tocopherol, β-tocopherol and tocotrienols; flavonoids such as quercetin and naringenin. Examples of the esters of the above acids include those having an alkyl ester group having 1 to 5 carbon atoms.

As for the stabilizing component to be mixed with the sanshools, one or more kinds may be used, or two or more kinds may be used. When two or more stabilizing components are used, it is preferable to use a water-soluble stabilizing component and a fat-soluble stabilizing component in combination.

### (Method for producing sanshool-containing composition)

The emulsifying step in the method for stabilizing sanshools of the present invention includes a step of adding a sanshool-stabilizing component to the sansho extract to obtain a sanshool-containing stabilizing component (step A); and a step of adding and mixing a solvent immiscible with the extract and an emulsifier to the sanshools containing the stabilizing component to obtain an emulsion (step B). Each of the sanshools and the stabilizing component may be dissolved or dispersed in the liquid. The solvent used in this emulsification step (step B) is not particularly limited, but examples thereof include edible oils, organic solvents, water, and liquid mixtures of water and organic solvents. In the step A of stabilizing the sanshools contained in the sansho extract, the liquid used in step A may be the extraction solvent used for extracting the sanshools from the sansho extract. Further, when stabilizing the sanshools obtained by synthesis, the liquid used in step A may be a solvent used during synthesis, such as a solvent used for purification. In step B, a solvent that is immiscible with the sanshool solution containing the stabilizing component obtained in step A is used.

The edible oil used as the solvent in the present invention is not limited among any edible oils that are usually eaten.

The organic solvent used in the present invention is not particularly limited, but examples include alcohols having 2 to 4 carbon atoms such as ethanol, 1-propanol, 2-propanol, 1-butanol and 2-butanol; ethers such as diethyl ether and tetrahydrofuran; esters such as ethyl acetate; ketones such as acetone; nitriles such as acetonitrile; aromatic hydrocarbons such as benzene and toluene; hexane; and mixture thereof. Among these, alcohols having 1 to 4 carbon atoms, esters, acetones and mixed liquids of one or more water-soluble organic solvents (described later) among these organic solvents and water are preferable; and alcohols having 1 to 4 carbon atoms, acetone, ethyl acetate, acetonitrile and mixed liquids of one or more water-soluble organic solvents (described later) among these organic solvents and water more preferable, ethanol, methanol, 2-propanol, acetone, ethyl acetate, acetonitrile, and mixed liquids of one or more of these organic solvents (excluding chloroform) and water are even more preferable.

In addition, when sanshools stabilized by emulsification of sanshools are used in foods, pharmaceuticals, or the like, it is preferable to use safe solvents such as ethanol or 1-propanol that can be used in foods and pharmaceuticals.

Sanshools tend to deteriorate over time. Therefore, step A for obtaining a sanshool-containing solution containing a stabilizing component is preferably carried out as soon as possible after extraction of sanshools from sansho or after synthesis of sanshools, and it is preferred to mix the sanshools and the stabilizing component in a liquid right after extraction or synthesis. For example, when producing drugs, supplementary foods, or the like containing sanshools, sanshools and stabilizing components are mixed in advance to stabilize the sanshools, and the sanshool solution containing the stabilizing components can be emulsified and mixed with materials that make up drugs and supplementary foods (products to which sanshools are applied), so that sanshools can be present in a stable state in these products and product life can be extended.

Step B in the present invention is a step of adding a solvent immiscible with the sanshools solution to which the stabilizing component has been added, and an emulsifier to emulsify the mixture by vigorously stirring.

As for the emulsifier to be used, in the case of o/w emulsion, an emulsifier with an HLB value of 6 or more and 17 or less is used, and in the case of w/o emulsion, an emulsifier with an HLB value of 7 or less is used. Needless to say, when the emulsifier is used for food, pharmaceuticals, or the like, it is limited to the approved emulsifier depending on the application.

Sanshools are presumed to be susceptible to hydrolysis under acidic conditions. It is conceivable that the stabilizing component of the present embodiment tends to be acidic when mixed with sanshools, depending on its type, amount of addition, conditions of addition, and the like. Therefore, when using a liquid containing at least one of water and a water-soluble organic solvent in the above step A or B, it is considered sanshools can be stabilized by mixing the sanshools and the stabilizing component under the condition that the pH is 6 or higher.

In the above step A or B, when a liquid containing at least one of water and a water-soluble organic solvent is used, the emulsification step is preferably carried out under conditions where the pH of the liquid is 6 or higher. It is more preferably 6.5 or more, and even more preferably 7.0 or more. The upper limit of the pH is not particularly limited, but is usually 14 or less, preferably 12 or less, more preferably 10 or less, and even more preferably 9 or less. When a liquid containing at least one of water and a water-soluble organic solvent is used, sanshools can be effectively stabilized by performing the mixing step under conditions of pH 6 or higher.

### (Powderization of emulsion of sanshools)

When an o/w emulsion, w/o emulsion, or w/o/w emulsion of sanshools is mixed with dextrin, lactose, crystalline cellulose, or precipitated silica having a DE value of 25 or more, and vacuum-dried or freeze-dried, a powder in which sanshools are stabilized can be obtained. Dextrin having a DE value of 25 or less causes destabilization of micelles constituting the emulsion, resulting in destabilization of sanshools. The mixing ratio of emulsion to excipients is 1:3 or more, and the amount of excipients may be increased. The preferred drying method is vacuum drying, in which case heating is not required. In the case of freeze-drying, sometimes stability problems arise due to the destruction of micelles during freezing. Since the amount equivalent to water is mainly removed from the powder during drying, the drying time should be determined based on the weight loss value.

### (Sanshool-containing composition)

The sanshool-containing composition of the present embodiment includes an o/w emulsion, a w/o emulsion, or a w/o/w emulsion containing a sanshool, a stabilizing component, an emulsifier, and a liquid, and a powdered emulsion thereof. Sanshools, stabilizing components, emulsifiers and liquids are as described above.

The pH of the sanshool-containing composition is 6 or higher, more preferably 6.5 or higher, and even more preferably 7.0 or higher. Although the upper limit of the pH is not particularly limited, it is usually 14 or less, preferably 12 or less, more preferably 10 or less, and even more preferably 9 or less. By setting the pH of the sanshool-containing composition to be 6 or higher, the sanshools can be effectively stabilized.

The content of the stabilizer contained in the sanshool-containing composition depends on the type of the stabilizing component, but it is usually preferably 0.2 mol or more per 1 mol of the sanshool, and more preferably 5 mol or more, and may be 1 mol or more, may be 5 mol or more, may be 10 mol or more, may be 15 mol or more, or may be 30 mol or less. may be 25 mol or less, or may be 20 mol or less. If the content of the stabilizing component for sanshools is too small, it tends to be difficult to stabilize the sanshools in the sanshools-containing composition.

Powder obtained by vacuum-drying or freeze-drying o/w emulsion, w/o emulsion, or w/o/w emulsion of sanshools mixed with dextrin, lactose, crystalline cellulose, or precipitated silica with a DE value of 25 or more can be used as a powdery sanshool-containing composition. Dextrin having a DE value of 25 or less causes destabilization of micelles constituting the emulsion, resulting in destabilization of sanshools. The mixing ratio of emulsion to excipients is 1:3 or more, and the amount of excipients may be increased. Vacuum-dried powders are preferred, and freeze-dried powders sometimes experience micelle disruption in the powder while freezing.

The sanshool-containing composition can be (a) as it is, (b) as particulate matter or powder after removing the liquid, or (c) as dissolved or dispersed the particulate matter or powder again in the liquid, added to various products such as foods, supplementary foods, beverages, pharmaceuticals, quasi-drugs, cosmetics and feeds. By using a sanshool-containing composition, the sanshools can be present in these products in a stable state, so it can be expected to improve the shelf time of these products.

### EXAMPLES

The present invention will be described in more detail below with reference to examples and comparative examples, but the present invention is not limited to these examples.

### [Analysis by high performance liquid chromatography (HPLC)]

High performance liquid chromatography (HPLC) analysis was performed on a reversed-phase system. The HPLC apparatus is LC-2010 (manufactured by Shimadzu Corporation), the pump is LC-10ADvp, the column oven is CTO- 10ACvp, and the system controller is SCL-10Avp. The column is Inert Sustain Swift C18 4.6 × 250 mm (GL Sciences), the column temperature is 40°C, the mobile phase is 30% acetonitrile for A liquid and 80% acetonitrile for B liquid, and the gradient conditions are 0 min B liquid 0% → 35 min B liquid 45 % → 50 min B liquid 100% → 51 min B liquid 0% → 55 min B liquid 0%, the detection wavelength was 270 nm, and the content of hydroxy-α-sanshool was calculated from the peak area value of hydroxy-α-sanshool.

### [Method of Conducting Stability Test]

After putting 4 ml of a sample into a 15 ml test tube with a cap and sealing it, it was incubated in a 50° C. oil bath. A sample was collected after a certain period of time, and a certain amount was dispersed in a constant volume of isopropyl alcohol, stirred, and hydroxy-α-sanshool was eluted in the isopropyl alcohol and analyzed by HPLC.

### [Manufacturing method of Sansho rapeseed oil extract]

8 g of rapeseed oil (FUJIFILM Wako Pure Chemical Industries, Ltd.) was added to 0.4 g of sansho powder, and after stirring, it was filtered by using a Buchner filter, Toyo Roshi Paper No. 1 to obtain a sansho rapeseed oil extract (hereinafter "S-NOX") by suction. A sansho rapeseed oil extract containing α-tocopherol (hereinafter "S-TOX") was also prepared by extracting sansho powder in the same manner as above using α-tocopherol-containing rapeseed oil in which α-tocopherol was added to rapeseed oil so that the final concentration was 1%.

### Example 1 Production of o/w emulsion

104 g of glycerin heated to 50° C was added to 10 g of various sucrose ester emulsifiers or polyglycerin ester emulsifiers having an HLB value of 6.5 to 17, and the mixture was stirred with a homogenizer at 8000 rpm for 1 minute. 32 ml of S-NOX or S-TOX was added and stirred again with the homogenizer at 8000 rpm for 5 minutes. Then, 48 ml of deionized water was added and stirred with a homogenizer at 8000 rpm for 1 minute. Then the prepared o/w emulsion was in a stable emulsified state even after standing at room temperature for 6 months or longer.

The stability of hydroxy-α-sanshool of this emulsion was investigated in an accelerated test at 50°C. Each emulsion was incubated at 50°C. Table 1 shows the residual ratio (%) on Day 3 and Day 7 when the amount of hydroxy-α-sanshool on Day 0 is taken as 100%. The o/w emulsions AT to FN are emulsions containing the following emulsifiers. The letter at the end indicates formulation (T) containing α-tocopherol as a stabilizing component and formulation (N) not containing α-tocopherol. Namely, (N) is indicated when S-NOX is used, (T) is indicated when S-TOX is used.
A... Sunsoft Q-17B (HLB: 6.5) (Taiyo Kagaku Co., Ltd.) diglyceryl mono-dioleate
B... Sunsoft Q-17S (HLB: 12.0) (Taiyo Kagaku Co., Ltd.) decaglycerin monooleate
C... Sunsoft A-171E (HLB: 13.0) (Taiyo Kagaku Co., Ltd.) pentaglycerol monooleate
D... Ryoto polyglyester L-7D (HLB: 17.0) (Mitsubishi Chemical Foods Co., Ltd.) decaglycerin laurate ester
E... Ryoto sugar ester S-1170 (HLB: 11.0) (Mitsubishi Chemical Foods Co., Ltd.) sucrose stearate ester
F... Ryoto sugar ester S-1670 (HLB: 16.0) (Mitsubishi Chemical Foods Co., Ltd.) sucrose stearate ester

As shown in Table 1, when the amount of hydroxy-α-sanshool on Day 0 is 100%, even in S-NOX or S-TOX, and in o / w emulsions made with various sucrose ester emulsifiers or polyglycerin ester emulsifiers, hydroxy-α-sanshool was found to be relatively stable. In addition, it was found that the use of S-TOX during preparation of the emulsion increased the stability of hydroxy-α-sanshool as compared to the use of S-NOX.

**[Table 1]**

| Residual ratio of hydroxy-α-sanshool in o/w emulsions | | | | |
|---|---|---|---|---|
| Liquid | | Day 0 (%) | Day 3 (%) | Day 7 (%) |
| o/w Emulsion | AT | 100 | 98.1 | 94.5 |
| | AN | 100 | 96.0 | 83.5 |
| | BT | 100 | 94.2 | 90.7 |
| | BN | 100 | 92.8 | 82.2 |
| | CT | 100 | 98.9 | 95.1 |
| | CN | 100 | 95.8 | 88.1 |
| | DT | 100 | 95.2 | 86.9 |
| | DN | 100 | 93.2 | 86.0 |
| | ET | 100 | 95.5 | 87.9 |
| | EN | 100 | 93.1 | 85.7 |
| | FT | 100 | 96.1 | 89.1 |
| | FN | 100 | 94.1 | 85.6 |
| 95%EtOH Extract | | 100 | 97.3 | 91.1 |
| Rapeseed Oil Extract (S-NOX) | | 100 | 99.1 | 94.6 |
| Rapeseed Oil Extract (S-TOX) | | 100 | 98.6 | 96.9 |

### [Preparation method of sansho ethanol extract]

8 ml of ethanol was added to 0.4 g of sansho powder, and after stirring, it was filtered by using a Buchner filter, Toyo filter paper No. 1 to obtain a sansho ethanol extract by suction.

### Example 2 Preparation of w/o emulsion

10 ml of sansho ethanol extract was diluted with 90 ml of deionized water. This solution was used as a diluent of ethanol extract of sansho (hereinafter "D-EtEX"). Instead of deionized water, a diluted solution of ethanol extract of sansho diluted with 10% aqueous sodium ascorbate solution (hereinafter "D-AsEX") was also prepared.

To 100 g of rapeseed oil heated to 60° C, 0.1 g of emulsifier of sucrose fatty acid ester type or polyglycerin ester type was added, and the mixture was stirred with a homogenizer at 8000 rpm for 1 minute. 10 ml of D-EtEX or D-AsEX was added and stirred again with the homogenizer at 8000 rpm for 5 minutes. It was found that the prepared w/o emulsion was in a stable emulsified state even after standing at room temperature for 6 months or more.

### Example 3 Preparation of w/o/w emulsion

To 10g of an aqueous phase emulsifier, Sansoft A-171E (HLB: 13.0) (Taiyo Kagaku Co., Ltd.) pentaglycerin monooleate, 104 g of glycerin heated to 50°C is added, and stirred and homogenized at 8000 rpm for 1 minute with a homogenizer. Add 32 ml of a w/o emulsion prepared by the method of Working Example 4 by using ryoto sugar ester ER-290 (HLB: 2.0) (Mitsubishi Chemical Foods Co., Ltd.) sucrose erucate ester as an oil phase emulsifier, and stirred again with a homogenizer at 8000 rpm for 5 minutes. Then, 48 ml of deionized water was added and stirred with a homogenizer at 8000 rpm for 1 minute. It was found that the prepared w/o/w emulsion was in a stable emulsified state even after standing at room temperature for 6 months or longer.

The stability of hydroxy-α-sanshool in these w/o/w emulsions was investigated in an accelerated 50°C test. W/o/w emulsions prepared from w/o emulsions made with D-EtEX or D-AsEX were incubated at 50°C. Table 2 shows the residual ratio (%) on Day 3 and Day 7 when the amount of hydroxy-α-sanshool on Day 0 is taken as 100%. As shown in Table 2, when the amount of hydroxy-α-sanshool on Day 0 is 100%, hydroxy-α-sanshool is relatively stable in any w/o/w emulsion prepared using the above w/o emulsion.

**[Table 2]**

| Residual ratio of hydroxy-α-sanshool in w/o/w emulsions | | | |
|---|---|---|---|
| Liquid | Day0 (%) | Day3 (%) | Day7 (%) |
| w/o/w Emulsion (made with D-AsEX) | 100 | 96.0 | 88.4 |
| w/o/w Emulsion (made with D-EtEX) | 100 | 92.5 | 86.9 |

### Example 4 Powderization of Sansho Rapeseed Oil Extract or Sansho Ethanol Extract

5 ml of sansho rapeseed oil extract or sansho ethanol extract was mixed with 20 g of lactose, dried in vacuum, and the obtained powder was subjected to an accelerated test at 50° C to determine the residual amount of hydroxy-α-sanshool. The powder was incubated at 50°C. Table 3 shows the remaining ratio (%) on Day 3 and Day 7 when the amount of hydroxy-α-sanshool on Day 0 is taken as 100%.

As shown in Table 3, it was found that hydroxy-α-sanshool was rapidly decomposed in any of the powders prepared using lactose, and was significantly decomposed on Day 7.

**[Table 3]**

| Residual ratio of hydroxy-α-sanshool in powdered sansho extract | | | |
|---|---|---|---|
| Powder | Day0 (%) | Day3 (%) | Day7 (%) |
| Powdered 95%EtOH Extract | 100 | 70.3 | 30.1 |
| Powdered Rapeseed Oil Extract | 100 | 97.1 | 68.7 |
| Powdered Rapeseed Oil Extract | 100 | 82.2 | 39.2 |

### Example 5 Powderization of o/w emulsion or w/o emulsion

5 g of the o/w emulsion prepared by using the S-NOX or S-TOX prepared in Example 1 and the w/o emulsion prepared using the D-EtEX or D-AsEX prepared in Example 2 were mixed with Pinedex #2 (DE value: 10 to 12, Matsutani Kagaku) or 20 g of lactose and the powder was obtained by vacuum drying.

Dextrin is obtained by chemically or enzymatically reducing the molecular weight of cornstarch or potato starch. A dextrose equivalent value (DE value) is used as an indicator of the molecular weight reduction. It is a relative measure when the reducing power of dextrose (glucose) is taken as 100, namely DE value of starch is 0. The range generally called dextrin has a DE value of 10 or less, the range of 10<DE value<20 is called maltodextrin, and the range of DE value>20 is called flour candy. The dextrin (DE value) used in this test is Pinedex #100 (4, Matsutani Chemical), Pineflow (7-12, Matsutani Chemical), Pinedex #2 (10-12, Matsutani Chemical), MARTRIN T180 ( 16.5-19.9, Matsutani Chemical) and MARTRIN M250 (23-27, Sansho Co., Ltd.). In addition, lactose (Fuji Film Wako Pure Chemical Industries, Ltd.) and crystalline cellulose (Merk) were also used.

When various dextrin or 30% lactose solutions was added to various emulsions, rapid disintegration of the emulsion and loss of cloudiness was observed if dextrin with a DE value of 25 or less was used. Table 4 shows the results for the o/w emulsion BN used in Example 1. 4 g of various sugars (1.6 g in case of lactose) were dissolved in 10 ml of deionized water, and 0.4 g of o/w emulsion was added thereto and stirred well, kept in room temperature, visually observed and evaluated according to the four-grade criteria shown in Table 5 below. According to the criteria in Table 5, the effect of various dextrins with DE value more than 25 on emulsion stability is evident. Moreover, destruction of micelles was observed by microscopic observation. On the other hand, in case of lactose, white turbidity persisted, and microscopic observation revealed no micelle destruction. It seems that the micelles are destroyed by the interaction between the many hydroxyl groups present in the hydrophilic part of the emulsifier and the hydroxyl groups of the dextrin sugar chain, on the contrary interaction is hard to occur in case of lactose as oligosaccharide.

**[Table 4]**

| Effects of powder on the stability of o/w emulsion | |
|---|---|
| Powder | o/w Emulsion |
| Pineflow (DE value: 7 ∼ 1 2) | △ |
| Pinedex # 2(DE value: 1 0 ∼ 1 2 ) | × |
| MALTRIN M250 (DE value: 2 3 ∼ 2 7) | ○ |
| Maltose(DE value: approx. 5 0) | ⊚ |
| Lactose(DE value: approx. 5 0) | ⊚ |
| Sucrose | ⊚ |

**[Table 5]**

| Evaluation Criteria | |
|---|---|
| ⊚ | Uniform cloudiness |
| **○** | Thin cloudiness |
| △ | Partially transparent |
| × | Completely separated |

Next, for the o/w emulsion powder, an accelerated test was conducted under the condition of 50° C by changing the type of powder, and the remaining amount of hydroxy-α-sanshool was examined. The powder was incubated at 50°C. Table 6 shows the residual ratio (%) on Day 3 and Day 7 when the amount of hydroxy-α-sanshool on Day 0 is taken as 100%. The letter at the end indicates a formulation (T) containing α-tocopherol as a stabilizing component and a formulation (N) not containing α-tocopherol.

As shown in Table 6, among the prepared o/w emulsion powder, the powder prepared using Pinedex #2, which is a dextrin with a DE value of 25 or less, showed hydroxy-α-sanshool was rapidly decomposed, but the powder prepared with lactose showed hydroxy-α-sanshool was stable. The similar results were also obtained with w/o and w/o/w emulsions.

**[Table 6]**

| Residual ratio of hydroxy-α-sanshool in powdered emulsion | | | |
|---|---|---|---|
| Powder | Day0 | Day3 | Day7 |
| Powdered o/w Emulsion (S-TOX) with Lactose | 100 | 94.5 | 92.2 |
| Powdered o/w Emulsion (S-NOX) with Lactose | 100 | 80.6 | 68.5 |
| Powdered o/w Emulsion (S-NOX) with Pinedex #2 (DE value:10-12) | 100 | 46.6 | 9.0 |

As described above, hydroxy-α-sanshool is present in ethanol or edible oil extracts of sansho, but hydroxy-α-sanshool degrades rapidly even if this solution is diluted or mixed with excipients to make powder. On the other hand, when these extracts are mixed with a component that stabilizes hydroxy-α-sanshool and emulsified with an emulsifier, the decomposition of hydroxy-α-sanshool can be prevented and it is possible to use it in liquid products. Moreover, when these emulsions are mixed with lactose, crystalline cellulose, etc. and pulverized, a powder in which hydroxy-α-sanshool is stabilized can be obtained. Mixing the emulsions with saccharide powder such as dextrin, soluble starch, results in destruction of the emulsion and is not suitable for powdered products. The Sansho extract powderized with lactose, crystalline cellulose, can be used for powdered products of Sansho because of stability.

## Claims

1. A method for stabilizing sanshools, the method including Step A and Step B below:
Step A: obtaining a sanshool solution containing stabilizing component by adding a stabilizing component of sanshools to a sanshool-containing liquid and;
Step B: obtaining an emulsion by adding, to the sanshool solution containing stabilizing component, an emulsifier and a solvent immiscible with the sanshool solution containing stabilizing component.

2. The method for stabilizing sanshools described in Claim 1, wherein
in Step A, an oil-soluble stabilizing component of sanshools is added to the oil extract of sansho to obtain a sanshool solution containing a stabilizing component, then in Step B, an emulsifier and water or a buffer solution are added to the said sanshool solution containing the stabilizing component to obtain an o/w emulsion.

3. The method for stabilizing sanshools described in Claim 1, wherein
in Step A, an ethanol-soluble stabilizing component or a water-soluble stabilizing component of sanshools is added to the ethanol extract of sansho to obtain a sanshool solution containing a stabilizing component,
then in Step B, an emulsifier and an edible oil are added to the said sanshool solution containing the stabilizing component to obtain an w/o emulsion.

4. The method for stabilizing sanshools described in Claim 1, wherein
in Step A, an ethanol-soluble stabilizing component or a water-soluble stabilizing component of sanshools is added to the ethanol extract of sansho to obtain a sanshool solution containing a stabilizing component,
then in Step B, an emulsifier and an edible oil are added to the said sanshool solution containing the stabilizing component to obtain an w/o emulsion, then an emulsifier which is the same with or different from the emulsifier described in Claim 2 and water or a buffer solution containing the stabilizing component to the w/o emulsion above to obtain an w/o/w emulsion.

5. The method for stabilizing sanshools including the steps wherein an emulsion obtained by the method for stabilizing sanshools described in any of Claims 1, 2, 3 or 4 is mixed with saccharide powder with a dextrose equivalent value 25 or more, insoluble polysaccharide, precipitated silica, or a combination thereof, and powdered by vacuum drying or freeze-drying.
